# EUROPEAN PATENT SPECIFICATION

(11) **EP 2 195 131 B1**
(45) Date of publication and mention of the grant of the patent: **26.10.2011**
(21) Application number: 08803417.8
(22) Date of filing: 29.08.2008
(51) Int. Cl.: B22F 3/22, B22F 7/00, C04B 35/63

(54) **METHOD FOR PRODUCING A THREE-DIMENSIONAL MACROPOROUS FILAMENT CONSTRUCT BASED ON PHASE INVERSION AND CONSTRUCT THEREBY OBTAINED**
VERFAHREN ZUR HERSTELLUNG EINER DREIDIMENSIONALEN MAKROPORÖSEN FILAMENTKONSTRUKTION AUF GRUNDLAGE VON PHASENUMKEHR UND DADURCH ERHALTENE KONSTRUKTION
PROCÉDÉ POUR LA PRODUCTION D'UNE CONSTRUCTION DE FILAMENT MACROPOREUX TRIDIMENSIONNEL BASÉ SUR L'INVERSION DE PHASE ET CONSTRUCTION OBTENUE SELON CE PROCÉDÉ

(30) Priority: 29.08.2007 EP 07115196
(43) Date of publication of application: 16.06.2010
(73) Proprietor: Vito NV, 2400 Mol (BE)
(72) Inventor: MULLENS, Steven Hans Rik Wouter, B-3271 Zichem (BE); THIJS, Ivo, B-2400 Mol (BE); LUYTEN, Jan Roger, B-3294 Molenstede (BE); BOUWEN, Wim Louis, B-2260 Westerlo (BE)
(74) Representative: Paemen, Liesbet R.J.
(86) International application number: PCT/EP2008/061429
(87) International publication number: WO 2009/027525

(56) References cited:
- EP-A1- 1 852 667
- EP-A1- 1 918 675
- EP-A2- 1 918 264
- EP-A8- 1 852 667
- EP-B1- 1 918 675
- WO-A-03/000480
- WO-A-2006/130935
- WO-A1-2008/006776
- WO-A2-2007/123725
- WO-A2-2008/002333
- MIRANDA ET AL: "Sintering and robocasting of beta-tricalcium phosphate scaffolds for orthopaedic applications" ACTA BIOMATERIALIA, ELSEVIER, AMSTERDAM, NL, vol. 2, no. 4, July 2006 (2006-07), pages 457-466, XP005489152 ISSN: 1742-7061
- SALGADO ANTONIO J ET AL: "Bone tissue engineering: State of the art and future trends" MACROMOL. BIOSCI.; MACROMOLECULAR BIOSCIENCE AUG 9 2004, vol. 4, no. 8, 9 August 2004 (2004-08-09), pages 743-765, XP002485214
- HUTMACHER D W ET AL: "Scaffold-based tissue engineering: rationale for computer-aided design and solid free-form fabrication systems" TRENDS IN BIOTECHNOLOGY, ELSEVIER PUBLICATIONS, CAMBRIDGE, GB, vol. 22, no. 7, July 2004 (2004-07), pages 354-362, XP004520508 ISSN: 0167-7799
- YEONG W-Y ET AL: "Rapid prototyping in tissue engineering: challenges and potential" TRENDS IN BIOTECHNOLOGY, ELSEVIER PUBLICATIONS, CAMBRIDGE, GB, vol. 22, no. 12, December 2004 (2004-12), pages 643-652, XP004637169 ISSN: 0167-7799
- OLIVIER V ET AL: "Biomaterial challenges and approaches to stem cell use in bone reconstructive surgery" DDT - DRUG DISCOVERY TODAY, ELSEVIER SCIENCE LTD, GB, vol. 9, no. 18, 15 September 2004 (2004-09-15), pages 803-811, XP004561932 ISSN: 1359-6446

## Description

### Field of the invention

The present invention relates to a method for producing a three-dimensional macroporous filament construct in which the filaments have an interconnected micro-porosity and rough surface topography. The method can be used for the synthesis of porous structures comprising fully micro-porous filament struts or filament struts having a microporous outer sheath. The method comprises a combination of a 3D filament deposition of a specific powder paste followed by a phase inversion step which provides an interconnected microporous morphology and surface roughness topography to the filament struts of the construct. The present invention can be used for the production of highly porous constructs which can be applied as orthopaedic implant material and tissue engineering matrices. The invention thus provides porosity on two levels: interconnected macroporosity with an adjustable pore size distribution between the filaments and an interconnected microporosity within the filamentary struts. The increased surface roughness makes such constructs osteoconductive and promote their osteoinductive behaviour. The method, described herein with specific reference to Ti-and Ti alloys, can in fact be used for all metals and also for ceramics and composite materials.

### Background

Highly porous constructs are of great importance for orthopaedic implants and bone tissue engineering. An interconnected pore structure with large, preferably >100 µm, pore sizes is prerequisite for bone ingrowths.

Different manufacturing routes have been applied to prepare polymeric, ceramic or metallic highly porous scaffolds with pore sizes ranging from 100 - 1000 µm.

Recent developments for the fabrication of tailored biocompatible metallic scaffolds using various foaming processes include for example, template methods mostly by coating polyurethane sponges with a powder suspension, as described in EP 1 362 129, direct foaming of a powder suspension, as described in EP 1 731 247, processing with space holders, as described in EP 1 523 390, chemical vapour deposition (CVD) on a carbon foam, as described in US patent N° 6,087,553, or Ti-Ni foam obtained by means of SHS technique (self propagating high temperature synthesis), as described in EP 1 663 330.

Recent developments for the fabrication of tailored biomedical scaffolds using various direct-write processes for solid free form (SFF) fabrication of periodic structures resulted in porous constructs based on extrusion-based robotic deposition, also referred as robocasting, as for instance disclosed in US patent N° 6,993,406 and US patent N° 6,401,795; three-dimensional printing using a 3D fiber depositing device, also referred as a Bioplotter, as for instance disclosed in EP 1 274 559, porous scaffold fabricated by rapid prototyping (Jia Ping Li et al. Biomaterials 27(2006) 1223-1235); Laser processing (Laoui & Santos, Proceedings of the Lane 2004, page 475-484), or Electron Beam Melting; as for instance disclosed in EP 1 583 628.

For example, Miranda et al. (Acta biomaterialia, 2, 457-466, 2006) disclose a method for making 3-D porous β-tricalcium phosphate (β-TCP) scaffolds using a robocasting (direct writing) technique. This document discloses the preparation of inks as a stable suspension of powder β-tricalcium phosphate in distilled water in the presence of dispersant and flocculant. The prepared powdery inks are then deposited directly in solvent environment, in particular in a non-wetting oil bath. Deposition in a solvent environment aims to avoid drying of the deposited inks.

In another example, international application WO 03/000480 discloses different casting methods for producing 3D structures, including solvent, melt, and slurry casting of polymers in pre-formed molds. For instance, the casting of polymer material in a pre-formed ceramic 3D mold is described. This document does not specifically mention particular conditions which need to be applied during formation of the molds or during casting of the polymer materials in the molds.

Besides the need for an interconnected pore structure with a high macroporosity and pore sizes preferably larger than 100 µm, also the morphology and more particular microporosity and surface roughness of the filament struts separating interconnected pores in a porous structure is extremely important.

A number of different methods for roughening the surface of dense implants are known, e.g. acid etching and blasting. Also, several coating techniques can be applied, based on e.g. sputtering, vapour deposition, laser deposition or spraying. The most widely used method involves the use of a titanium plasma spray (TPS). TPS both roughens and coats the implant. However, TPS, like most other coating or roughening techniques, is a line-of-sight technique that is only applicable to dense surfaces as the interior of porous substrates can not be reached. As such, only the exterior is affected, without changing the interior of porous substrates.

In view of the above, it is clear that there is a need in the art for providing porous filamentary implant constructs having improved filament roughness as well as improved (micro)porosity. So far, according to the Applicants best knowledge, techniques enabling to prepare constructs having a desired surface roughness together with a desired porosity, especially micro-porosity, have not been reported.

The present invention aims to provide a porous filamentary construct having improved filament morphology. The present invention seeks to remedy at least some of the aforementioned problems and drawbacks of the currently available constructs, and offers other advantages over the prior art. The invention aims to provide a method for preparing a three-dimensional macroporous filament construct made of filaments combining interconnected and controlled microporosity and rough surface topography.

The present invention also aims to provide a three-dimensional macroporous filament construct in which a desired rough surface topography is combined with a controlled microporosity.

### Summary

The present invention thereto provides a method as defined in claim 1 for producing a three-dimensional macroporous filament construct having a controllable microporous filament morphology which is induced by phase inversion. The method can be used for the preparation of a porous structure comprising fully microporous filament struts, filament struts having a microporous sheath, or filament struts having a microporosity that varies from the inside to the outside of the filament.

The present invention consists of a combination of a 3D filament deposition of a specific powder pasta followed by a phase inversion step creating an improved and desired morphology and topography to the filament struts of the structure. The present invention can be used for the production of highly porous constructs which can be applied as orthopaedic implant material and tissue engineering matrices. The highly porous structures are characterized by a macroporosity with an adjustable pore size distribution between the filaments and an interconnected microporosity in the filaments themselves, making these materials suitable to optimize osteoinduction and osteoconduction.

The method, described herein for Ti-and Ti alloys, can be used for all metals and even for ceramics and composite materials.

More in particular, in a first aspect the present invention provides a method for producing a three-dimensional macroporous filament construct comprising filaments having an interconnected microporosity and a suitable morphology as defined in claim 1.

In a preferred embodiment, step b) is carried out in a non-solvent environment.

In yet another preferred embodiment, step c) comprises the steps of:
c1) inducing phase inversion by bringing said filaments during the deposition of the filaments into contact with a non-solvent vapour, and
c2) completing phase inversion by immersing the structure of step c1) in a liquid non-solvent, thereby creating a filament-based porous structure having suitable filament morphology.

Inducing phase inversion already during deposition of the filaments (step c1) of the present method) permits to create filaments that have sufficient rigidity and strength in order to give a solid 3D structure. The external layer of the filaments will become rigid during this step. Advantages thereof include the fact that bridges formed by the filaments in the 3D structure do not sag and that a good adhesion is obtained between filaments making contact with one another. Step c1) of the present method further permits to create filaments that have a suitable surface roughness by phase inversion at the surface.

Phase inversion is completed in a next step (step c2) of the present method) by immersing the structure in a liquid non-solvent. Omitting step c1) would result in filaments that do not adhere to each other and would not provide suitable 3D structures. Step c2) of the present method further permits to create filaments that have a suitable interconnected microporosity by completing the phase inversion in the bulk of the filament.

The filaments in the sintered filament-based porous structure obtained after step d) comprise an average surface roughness (Ra) which is higher than 4µm. Moreover, the filaments in the sintered filament-based porous structure obtained after step d) also have a microporosity (after sintering) comprised between 1 and 50%, preferably between 5 and 30%. In other words, the invention provides a method wherein the filaments in the filament-based porous structure have between 1 and 50%, preferably of between 5 and 30% of micropores, wherein said micropores consist of pores having a pore size equal to or smaller than 100µm.

Application of a phase inversion technique according to the present invention provides a suitable microporosity to the filaments which
1) is interconnected as a result of the phase inversion process whereby solvent and non-solvent are exchanged,
2) is controllable with regard to pore size and quantity, and
3) can be provided at a specific location in the filament, e.g. in the external layer.

In view of the combination of these three features, phase inversion is to be considered as a novel technique to provide a suitable microporosity in ceramic or metallic materials.

In a second aspect, the invention provides a three-dimensional macroporous filament construct as defined in claim 15 comprising filaments which have interconnected microporosity and which have a suitable morphology, said construct being obtainable by carrying out the present method. More in particular, the invention provides a three-dimensional macroporous filament construct comprising filaments which have between 1 and 50%, preferably between 5 and 30% of interconnected micropores, wherein said micropores consist of pores having a pore size equal to or smaller than 100µm, and which filaments have an average surface roughness (Ra) higher than 4µm, whereby said construct is obtainable by the method according to the present invention.

The present invention also provides a three-dimensional macroporous filament construct for adhering a bone promoting protein (BMP), stem cells, osteoblast cells, pharmaceuticals or/and a mixture thereof made of a predetermined material selected from the group comprising metal, ceramic or composite materials, and preferably Ti or a Ti alloy, comprising filaments having an average surface roughness (Ra) which is higher than 4µm. Preferably filaments in said construct have interconnected microporosity whereby said microporosity is between 1 and 50%, preferably between 5 and 30%. The present construct is thus characterised in that it comprises filaments having an average surface roughness (Ra) which is higher than 4µm and having between 1 and 50%, preferably between 5 and 30% of interconnected micropores, wherein said micropores consist of pores having a pore size equal to or smaller than 100µm. It shall be stressed that the present structure is in particular characterised in that it has *both* a surface roughness of higher than 4µm and a microporosity of between 1 and 50%. Having regard to the prior art in the present technical field, such construct can be considered as unconventional and unusual, especially since the prior art in this field does not report the possibility of having both of these parameters of surface roughness and microporosity in a single construct.

Unexpectedly the present invention permits to provide a three-dimensional macroporous filament construct having both parameters in combination in one scaffold. Further these two parameters reflect the improvements that are obtained with the present method. Thus the features of the present construct are linked to the inventive idea of applying a phase inversion technique, i.e. by exposing said filaments during the deposition of the filaments to a non-solvent vapour and to a liquid non-solvent. Structures having both indicated parameters can to the Applicants best knowledge not be fabricated using known fabrication techniques.

In a third aspect, the invention provides a composition which comprises a construct as disclosed herein and further comprises a bone promoting protein (BMP), stem cells, osteoblast cells, pharmaceuticals or/and a mixture thereof.

In a fourth aspect, the invention relates to the use of a construct or a composition as disclosed herein for the manufacture of a biomedical product, such as a synthetic bone implant or bone graft, a tissue engineering scaffold, a drug-delivery device.

In a fifth aspect, the invention therefore also provides a biomedical product such as a synthetic bone implant or bone graft, a tissue engineering scaffold, a drug-delivery device comprising a construct or a composition as disclosed herein.

Phase inverted filamentary constructs (PIFC) produced in accordance with the present method have filament average surface roughness (Ra) which is higher than 3 µm, and preferably higher than 4 µm. Based on specific processing parameters the microporosity induced within the filaments after sintering is associated with the obtained surface roughness in the present invention and can be finger-like, sponge-type or a combination of finger-sponge type. A roughened surface facilitates osseointegration of an implant and surrounding bone and also aids in mechanical retention of an implant at the time of placement. Surface roughness also modulates the activity of cells interacting with an implant, and thereby affects tissue healing and implant success. In addition, the surface topography of an implant plays a critical role in the bone cell response *in vitro* and *in vivo.* Osteoblast proliferation and differentiation and matrix production *in vitro* has been shown to be affected by the surface topography of titanium. When titanium hip stems are implanted *in vivo*, surface topography affects the attachment rate and strength of the bone-implant bond. Smooth surfaces of titanium induce fibrous tissue, whereas rough surfaces induce bone formation. The type of tissue formation affects both the regenerated tissue quality and the strength of the tissue-implant bond. The strength of tissue-implant bond plays a major role in the clinical success of the implant.

Phase inverted filamentary constructs (PIFC) produced in accordance with the present method also have a high available surface area, which is approximately increased with a factor of at least 2 or more compared to the surface available on prior art constructs, e.g. on non-phase inverted constructs. The available surface area has an impact on the number of adherent cells and stability of the construct-bone interface. The specific surface area and morphology also has an impact on coating and cell attachment for therapeutic and regenerative activity immobilization.

Besides the macroporosity of said construct which originate from the pores between the filaments, phase inverted filamentary constructs (PIFC) produced in accordance with the present method also have a high degree of interconnected microporosity within the filaments. The microporosity induced in the filaments after sintering is a combination of (1) the porosity directly induced by the displacement of solvent and non-solvent during the phase inversion process, (2) the calcination of the solidified phase inversion polymer and (3) the shrinkage of the pores during sintering. Said filament (micro)porosity is between 1 and 50 %, preferably between 5 and 30%. In particular, said filament has between 1 and 50 %, preferably between 5 and 30% of micropores, wherein said micropores consists of pores having a pore size of less than 100µm. The invention thus provides porosity on two levels: interconnected macroporosity with an adjustable pore size distribution between the filaments and an interconnected microporosity within the filamentary struts. Interconnected micropores within the filaments provides paths for nutrient transfer and signalling molecules to sustain the growing host bone and facilitate osteoinduction and osteoconduction.

Especially when used as a biomedical product, such as a synthetic bone implant or bone graft, a tissue engineering scaffold, or a drug-delivery device, a filament-based construct according to the invention comprising filaments with a suitable interconnected microporosity has the advantages of providing:
1) improved perfusion and improved nutrient transport through the construct;
2) a higher coefficient of friction and less micromotion of the construct (implant) to the surrounding (bone) tissue; a high degree of micromotion, being the relative motion at the bone-implant interface, is recognized as a factor which decreases bone ingrowth and results in the formation of fibrous tissue. The friction coefficient influences the mechanical stability of the implant and its relative motion with the surrounding bone.
3) better cell differentiation and formation of real bone tissue instead of fibrous tissue
4) better cell attachment and
5) easier application of additional coating(s) on the filaments.

The above-indicated morphology of the obtained construct, and in particular, the surface roughness and microporosity of the filaments, surface availability of the construct, determines a.o. the wicking speed, which has an influence on the transport of nutrients to the interior of the scaffold. In addition, the present method yields constructs with a higher coefficient of friction, and a lower elastic modulus, which will have an influence on the stability of the structure bone interface.

In view of the above, it is clear that the present construct has characteristics which makes it particularly suitable for being used as orthopaedic implant material and/or tissue engineering matrix. The invention thus provides highly porous constructs having an adjustable pore size distribution between the filaments and filamentary struts with controllable and interconnected microporosity and increased surface roughness; which makes such constructs osteoconductive and promote their osteoinductive behaviour.

Other benefits, advantages and uses of the invention will become apparent upon reading and understanding the below given specification and accompanying drawings.

### Description of the drawings

**Fig. 1** represents a flowchart of a general method according to the present invention.
**Fig. 2** shows a Ti-porous scaffold with a detailed image (profilometer) of the filament surface pattern without (A) and with (B) phase inversion using polysulphone PSf as a binder.
**Fig. 3** shows a Ti-porous scaffold with a detailed image (SEM) of the filament morphology and interconnected microporosity with (A) and without (B) phase inversion using polysulphone PSf as a binder.
**Fig. 4** shows SEM images of cross-sections of filaments obtained with variation in % polysulphone binder (wt% polysulphone PSf on Ti) from 0.00% (A); 2.78 % (B), 2.96 %(C), 4.72% (D), 8.7% (E) and 11.39% (F) according to example 2 of the present invention.
**Fig. 5** shows a SEM image of a cross-section of a filament obtained with phase inversion using a cellulose acetate binder according to example 3 of the present invention.
**Fig. 6(A)** shows a co-extrusion nozzle 1 suitable for being used in accordance with the present method. **Fig. 6(B)** shows a perspective view of different nozzles 4 which can be used in accordance with the present method. Using the illustrated nozzles 4 in a method according to the invention permits to form profiled filaments. **Fig. 6****(C**) shows a perspective view of different filaments obtained by extrusion through nozzles as illustrated on FIG. 6B.
**Fig. 7** shows microscopic images of (A) a section of a co-extruded construct and (B) a cross section of a single filament therein.
**Fig. 8** shows an example of a titanium macroporous filament construct obtained with a method according to the present invention.
**Fig. 9** shows an example of a hydroxyapatite macroporous filament construct obtained with a method according to the present invention.
**Fig. 10** illustrates the influence of the amount of a polysulphone phase inversion binder (in wt % PSf) on the surface roughness (Ra) of filaments in a construct according to the invention.
**Fig. 11** illustrates the influence of the amount of a polysulphone phase inversion binder (in wt% PSf) on microporosity (in %) of filaments in a construct according to the invention.
**Fig. 12** illustrates the influence of the amount of a polysulphone phase inversion binder (in wt% PSf) on the specific surface area (SSA in m²/g) of filaments in a construct according to the invention.
**Fig. 13** illustrates the influence of the amount of a polysulphone phase inversion binder (in wt% PSf) on ductility (in %) of filaments in a construct according to the invention.

### Detailed description of the invention

The present invention provides a method based on phase inversion for manufacturing a three-dimensional macroporous construct having a suitable microporous filament morphology. The sintered construct obtained with a method according to the invention comprises filament struts with an interconnected microporous structure and roughened surfaces.

The term "*phase inversion*" as used herein involves the process of transforming a polymer, e.g. deposited in the form of filaments, in a controlled manner from a liquid to a solid state. This process comprises the making of a suspension or a solution of a predetermined material to become the construct in a suitable solvent/phase inversion binder mixture, forming the suspension or solution into a desired shape, and exposing the suspension or solution to a non-solvent to cause the phase inversion binder to precipitate from the suspension or solution and form a construct in the desired shape. The phase inversion technique involves the precipitation or solidification of the phase inversion binder in a non-solvent, so that a solid matrix is formed. The term "phase inversion" is well known in the art of producing porous constructs and for instance clearly defined in the textbook entitled "Basic Principles of membrane Technology", from Mulder M., Kluwer Academic Publishers, 1996.

The term "*porosity*" as used herein refers to a measure of the void spaces in a material after sintering, and is measured herein as percent between 0 and 100%.

The terms "*macroporosity*" or "*macroporous*" or "*highly porous*" as used herein refer to porosity of the construct with macropores having a pore size greater than 100 µm in diameter. The terms "macroporosity" or "macropores" are in some embodiments of the invention considered as synonyms. The term macroporosity thus indicates that the construct of the invention has macropores having a pore size greater than 100µm in diameter.

The term "*microporosity*" or "*microporous*" refers to porosity of the filaments as such after sintering and includes micropores having a pore size equal to or smaller than 100 µm. The terms "microporosity" or "micropores" are in some embodiments of the invention considered as synonyms. The term microporosity thus indicates that the filaments of the invention have micropores having a pore size equal to or smaller than 100 µm in diameter, and for instance having a pore size comprised between 0.1 and 100µm, or between 0.5 and 100µm, or between 1 and 30µm, and for instance lower than 90, 80, 70, 60, 50, 40, 30, 20, 15, 10, 5, 1 µm.

The terms "*strut filaments*", "*struts*" or "*filaments*"" are used herein as synonyms and refer to suspension or solution, as defined herein, that has been deposited, e.g. by (co)extrusion, in the form of filaments.

The term "*interconnected*" microporosity is used herein in reference to microporous filaments and indicates that the (micro)pores of various filaments which are deposited according to the invention in a predetermined 3D pattern are in connection with each other, and as such provide paths, e.g. for nutrient transfer and signalling molecules, within the construct, and between filaments according to the invention

The term "*interconnected*" macroporosity is used herein in reference to the macroporous construct and the (macro)pores of the construct are in connection with each other, and as such provide paths, e.g. for nutrient transfer and signalling molecules, within the construct according to the invention.

The term "*roughening*" and its cognates are intended to refer to surface texture on the order of microns. Surface roughening is not intended to refer to larger features of an implant such as the thread.

The term "*surface roughness*" as used herein is also defined by the norm ISO 4287-1:1984 (Surface roughness -- Terminology -- Part 1: Surface and its parameters). Surface roughness can for instance be measured by non-contact, optical profilometry based on interferometry (VEECO, Wyko NT3300 - A.G. Olszak, J. Schmit, M.G. Heaton, "Interferometry: Technology and Applications," Veeco Instruments, Inc., 2650 E. Elvira Road, Tucson, AZ 85706, 2001).

It shall be noted that in some embodiments of the present invention, the terms "construct" and "structure" and "scaffold" are used as synonyms. In some embodiments of the present invention, the terms "suspension", "solution" and "paste" are used as synonyms.

### A. Method

The invention provides a method for producing a three-dimensional macroporous filament construct comprising interconnected microporous filaments having a desired morphology.

### STEP a)

A first step in such method comprises preparing one or more suspensions or pastes comprising particles of a predetermined material, a liquid solvent, one or more binders and one or more dispersants.

Predetermined materials that can be used include but are not limited to ceramics, such as alumina, mullite, zirconia, silicon carbide, silicon nitride, zinc oxide, barium titanate, barium strontium titanate, lead zirconate titanate (PZT), kaolin, calcium phosphates, hexaaluminates; metals and alloys, such as titanium, titanium-6aluminium-4vanadium, tantalum, tungsten, silver, molybdenum, and stainless steel; polymers, thick-film pastes, epoxies, sol-gel materials, and composite materials, such as Al₂O₃/TiCuSil, Al₂O₃/Al, Al₂O₃/Mo, zirconia/mullite, porous/dense PZT materials, porous/dense alumina, and PZT/polymer materials. In a preferred embodiment said predetermined material is a metal, ceramic or composite material. In a particularly preferred embodiment, said predetermined material is Ti or a Ti-alloy, preferably in powder form. Below the method is described in reference to the use of Ti-and Ti alloys as predetermined material. However, it shall be clear that the present method can be used for all metals and even for ceramics and composite materials, such as those given above.

The particles are preferably provided in powder form. The particles of said predetermined material preferably have a particle size of less than 100 µm, and more for instance of between 1 and 10µm, between 1 and 20µm, between 10 and 50µm or between 1 and 100 µm.

The present suspension preferably comprises between 20 and 90 wt%, and preferably between 25 and 85 wt%, and for instance between 50 and 85 wt% of predetermined material.

The invention provides a method wherein said liquid solvent is a nonvolatile liquid, a volatile liquid or a mixture thereof. Suitable solvents include but are not limited to N-methyl-pyrrolidone (NMP), dimethyl-acetamide (DMAc), dimethylformamide (DMF), dimethylsulphoxide (DMSO), tetrahydrofurane (THF), s-caprolactam, 4-butyrolactone, methyl ethylketone, acetone, acetic acid, formylpiperidine, morfoline, chloroform and dioxane. Preferably said nonvolatile liquid is N-methyl-2-pyrroliodone, and said volatile liquid is acetone.

In a preferred embodiment, said liquid solvent is soluble or miscible into a non-solvent as defined herein. The non-solvent as defined herein may comprise a non-solvent in a liquid or vapour phase. The liquid non-solvent as used herein is intended to comprise water, an alcohol, an acid, or mixture thereof. The liquid non-solvent can be used in a mixture with said solvent (see below).

In a preferred embodiment, said binder comprises a mixture of one or more phase-inversion binder(s) and one or more rheology modification binder(s).

The term "*phase inversion binder*" as used herein refers to a binder which is capable of allowing phase inversion in a suitable non-solvent medium e.g. water, an alcohol, an acid, or a mixture thereof or in a suitable medium comprising a mixture of a non-solvent and a solvent (see below). According to the present invention, the phase-inversion binder preferably comprises one or more polymers selected from the group comprising polysulphones, polyethersulphone, cellulose acetate, polyvinylidenefluorides, polyacrylonitriles, polyethylene-co-vinylalcohol, polyimides, polyether imides, polyamides and/or combinations thereof.

The term "*rheology modification binder*" as used herein refers to a binder which is capable of modifying the rheology or flow properties of the suspension as defined herein. According to the present invention, the rheology modification binder is preferably chosen from the group comprising hydrocolloids, cellulose derivates and/or combinations thereof.

In a particularly preferred embodiment, the present suspension comprises a total amount of binder, i.e. phase-inversion binder and rheology modification binder, of between 1 and 30 vol%, and preferably between 3% and 30 vol%, and for instance 5, 7, 10, 12, 15, 18, 20, 22, 25, 28 % depending of the kind of the powder, e.g. its density, particle size, specific surface. In another preferred embodiment, a suspension as defined herein comprises between 2 and 30 %, and preferably between 2 and 15 % of a phase-inversion binder and between 1 and 30 %, and preferably between 1 and 15 % of a rheology modification binder.

In another embodiment, the invention provides a suspension comprising one or more dispersants. Useful dispersants include but are not limited to darvan, targon, triton. Preferably, said dispersants are applied in an amount of between 0.01 and 5 %, and preferably of between 0.5 and 3 %.

The total volume % of the organic part of the suspension depends on the density, the particle size distribution, the morphology and the specific surface of the powder particles. Preferably, the composition of the paste is developed in order to minimize ash residue after thermal treatment. Preferably less than 6 weight % of rheology modification binder, plasticizer and/or dispersant is added.

The term "plasticizer" as used herein refers to one or more organic additives which are suitable for improving flexibility and plasticity of the suspension. Such plasticizers may include but are not limited to phtalates such as, dibutyl phthalate, dihexyl phthalate, triphenyl phthalate, (dipropylene glycol) butyl ether, diethyleneglycolmonoricinoleate, a natural or synthetic wax selected from the group comprising cetyl alcohol, stearylalcohol, cetostearylalcohol, bees wax, candellila wax, shellac wax, carnauba wax, or petroleum wax or a mixture thereof, and/or combinations thereof. Preferably the plasticizers are applied at an amount lower than 10 wt%, and preferably lower then 5 wt%.

In an example, a powder paste can be prepared by mixing 20 to 85 wt% of a Ti- or Ti-alloy powder with binders, plasticizers and dispersants in a solvent. In another example, a suspension mixture that is extruded according to the present invention comprises 1 to 30 vol% of organic material, 40 to 85 wt% of predetermined material, the remainder being solvent. The term "organic material" as used herein refers to additives which are added to the predetermined material and is intended to include binder(s), plasticizer(s), and dispersant(s).

In another preferred embodiment, the present suspension has a viscosity of between 100 and 10000 Pa s at a shear rate of 1 s⁻¹ and a yield point between 100-1000 Pa.

In a preferred embodiment, the invention provides for the preparation of different types of suspensions. For instance, preparation may involve:
A) the preparation of "*phase-inversion suspension*", i.e. a suspensions capable of undergoing phase inversion in a suitable non-solvent medium, comprising a predetermined material, a liquid solvent, one or more phase-inversion binder(s), one or more rheology modification binder(s), and one or more dispersants, and/or
B) the preparation of a "*phase-inversion suspension*" as in A but with a lower amount, preferably a 10, 20, 30, 40, 50% lower amount of phase inversion binder than in the suspension under A, and/or
C) the preparation of a "*non phase-inversion suspension*", i.e. a suspension not capable of undergoing phase inversion in a suitable non-solvent medium, comprising suspensions as defined herein, but lacking a phase-inversion binder.

The above indicated suspensions can advantageously be used for depositing filaments in accordance with the present invention using a co-extrusion method (see under method step b).

### STEP b)

A next step in the present method comprises depositing a suspension as defined herein in the form of filaments in a predetermined three-dimensional pattern thereby creating a three-dimensional filament-based porous structure. Preferably, the suspension is deposited in a non-solvent environment. Deposition of said suspension in the form of filaments can be performed by means of a direct writing method, a rapid prototyping method, a dispensing method or an extrusion method.

The term "*non-solvent environmenf*" as used herein refers to an environment with a relative non-solvent vapour of at least 10%, and preferably of at least 15, 20 or 25%

In a preferred embodiment, said non-solvent environment is a humidifying environment or ambient air with a relative humidity at least 50 %.

In a more preferred embodiment, said non-solvent environment is created by a gas stream of water vapour.

As mentioned above, deposition of the prepared suspension in the form of filaments can be performed by means of different techniques, including extrusion of the prepared suspensions through nozzle(s).

According to the invention, different types and configurations of nozzles can be used here for.

A nozzle 4 or nozzle head 4 according to the present invention can be defined as a body 4, which can be connected to a nozzle tube 6. The nozzle 4 is characterised by an outer wall 12 of which the inner surface 7 may be profiled or non-profiled.

In one embodiment, deposition of said suspension in the form of filaments is performed by extrusion of said suspension through a nozzle having a non-profiled inner surface. The term "*non-profiled inner surface*" indicates that the nozzles for use in an extrusion method according to the invention have a relatively smooth inner surface, free of indents, grooves or similar structures.

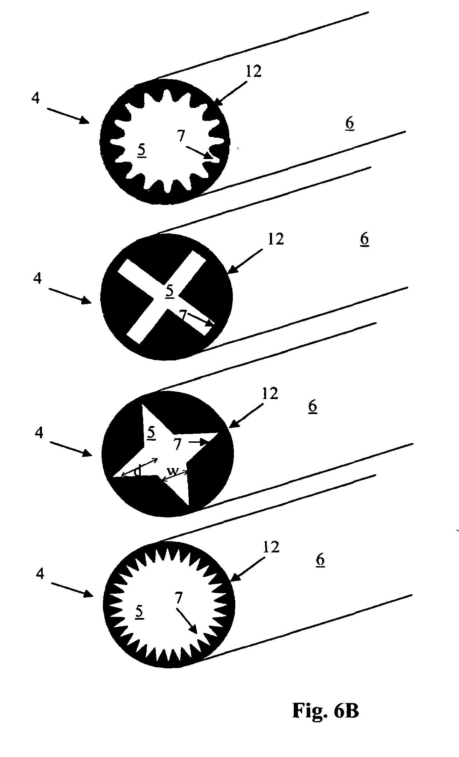

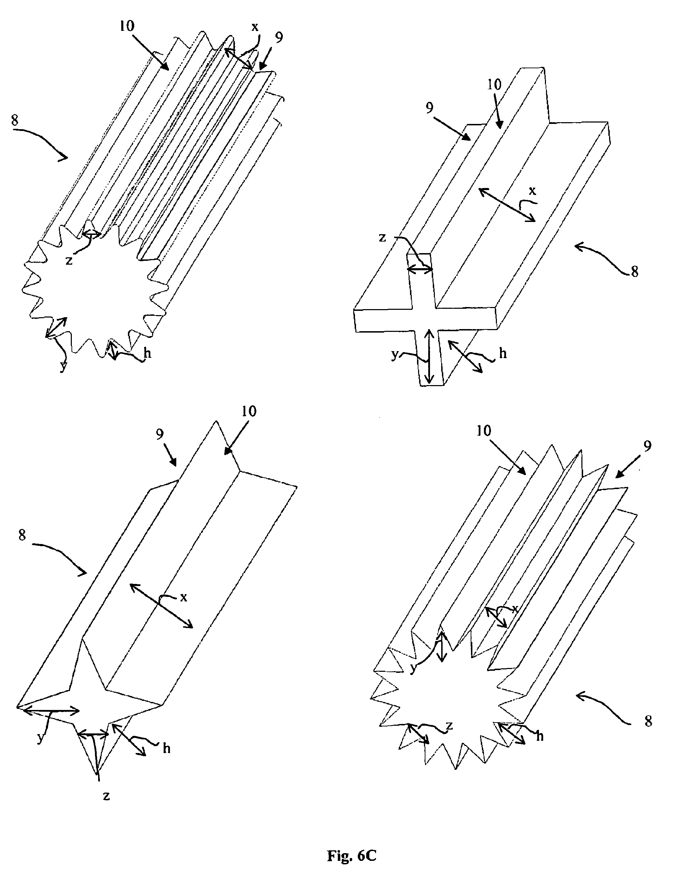

In another embodiment, deposition of said suspension in the form of filaments is performed by extrusion of said suspension through a nozzle having a profiled inner surface. The term "profiled inner surface" or "profiled nozzle" refers to nozzles that have an inner surface which is provided with geometric structures, such as e.g. indents, grooves, lines or other structures. In other words the outer wall of said nozzles has an inner surface showing geometric structures. **Fig. 6 (B)** shows a few examples of profiled nozzles 4 which can be used in accordance with the present method. The illustrated nozzles 4 are attached to a nozzle tube 6 and are provided with profiles or structures such as grooves and/or ribs on their inner surface 7. Reference number 5 refers to the aperture by which a suspension as defined herein leaves a nozzle 4. Using the illustrated nozzles 4 in a method according to the invention filaments 8 showing geometric structures such as e.g. grooves 9 and ribs 10 can be made, such as those illustrated on FIG. 6C.

In a preferred embodiment, profiled or structured nozzles have geometric structures, e.g. show grooves or similar structures, that have a depth (d) preferably of between 0.1 and 50µm, and for instance of between 10 and 50µm, and a width (w) of between 0.1 and 50µm, and for instance of between 10 and 50µm.

In another preferred embodiment, using such profiled or structured nozzles, profiled filaments 8 can be made, as illustrated on FIG. 6C, which show grooves 9 and ribs 10. Using profiled nozzles permits to deposit "profiled filaments", i.e. filaments showing a geometric pattern or a geometric structures such as grooves and ribs or similar structures on their surface e.g. running in the longitudinal and/or radial direction. Profiled filaments have the advantage that besides a defined surface roughness and microporosity, they also advantageously have geometric roughness. "Geometric roughness" can be defined in accordance with the present invention as roughness that follows a certain geometric pattern or structure, e.g. in the longitudinal and/or radial direction, such as for instance ribs, grooves, indents or the like. Constructs prepared based on profiled filaments, combining the above-indicated features of surface roughness, microporosity, and geometric roughness have improved binding/adhesion properties for living tissues and/or cells.

In a preferred embodiment, the present invention provides constructs having profiled filaments 8, wherein said filaments have grooves 9 that have a height (h) of between 0.5 and 50µm, and for instance of between 1 and 25µm, and a width (x) of between 0.5 and 50µm, and for instance of between 1 and 25µm.

In another preferred embodiment, the present invention provides constructs having profiled filaments 8, wherein said filaments have ribs 10 that have a height (y) of between 0.5 and 50µm, and for instance of between 1 and 25µm and a width (z) of between 0.1 and 50µm, and for instance of between 1 and 25µm.

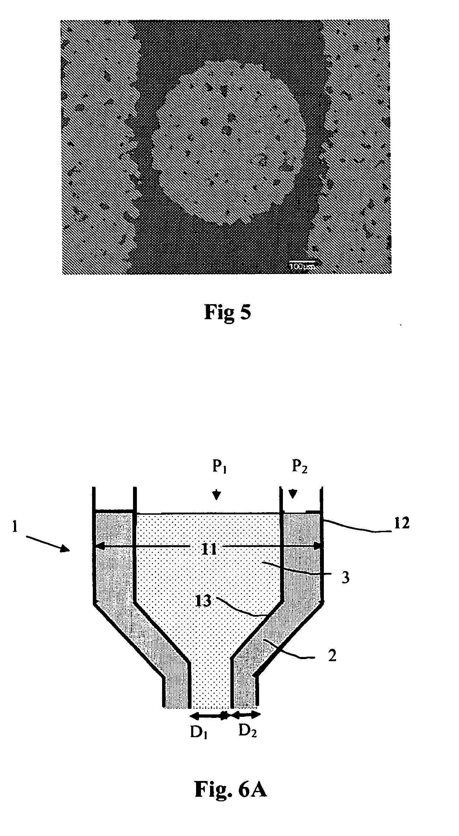

In another embodiment, the invention provides a method wherein deposition of said suspension in the form of filaments is performed by co-extrusion of suspensions having different compositions through separated inner and outer parts of a nozzle, thereby forming filaments showing an internal zone and an outer sheath. Co-extrusion is advantageous in the case of applications where high mechanical strength is required; co-extrusion with a combination of two or more different suspensions into a single filament structure can then be performed. In such embodiment, a porosity gradient within the filament diameter can be created by using two or more different concentrations of phase inversion binders in the different suspensions. Preferably, the outer sheath of a filament is obtained by extrusion of a suspension containing a higher concentration of phase inversion binders, leading to an increased microporosity in the outer sheath. The invention thus provides a method wherein the amount of said micropores in said filaments is different in said internal zone and in said outer sheath. Preferably the invention provides a method for producing a construct wherein the filaments have between 1 and 50%, and preferably between 5 and 30% micropores in the outer sheath, and between 1 and 10%, and preferably between 1 and 5% micropores in the internal zone. Lowering the concentration of phase inversion binders in the suspension that is extruded through the internal part of a nozzle leads to a lower amount of micropores in the core of the filaments. The type of nozzles that can be used in this kind of application is for instance illustrated on FIG. 6A.

In such embodiment, different suspensions can be applied, comprising at least one or more *phase-inversion suspensions* and optionally a *non phase-inversion suspension.* Using a co-extrusion technique, the microporosity of said filaments is different in an internal zone and an outer sheath. The method provides the possibility to create a predefined microporous filament sheath. In a preferred embodiment, the method comprises the deposition of filaments wherein the ratio of the diameters of the outer layer and the inner layer of a filament can be varied.

The suspensions indicated under step a) above can advantageously be used for depositing filaments in accordance with the present invention using a co-extrusion method. The filaments can be deposited such that the internal zone of the filaments is made of a suspension containing no or less phase inversion binder (e.g. suspension B or C), and the sheath of the filaments is made of a suspension containing phase inversion binders (e.g. suspension A).

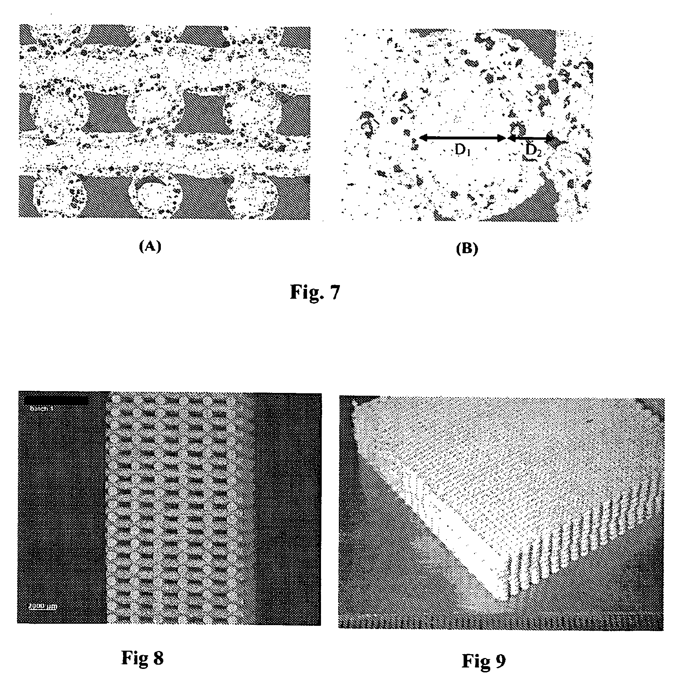

In a preferred embodiment, the internal zone of said filament is obtained by extrusion of a non phase inversion suspension which is not able to undergo phase inversion and the outer sheath of the filament is obtained by extrusion of a phase inversion suspension which is able to undergo phase inversion. In such embodiment, the internal zone has no microporosity, the microporous structure is created at the surface of the filaments. Referring to **FIG. 6A** a nozzle is illustrated which can be used in a co-extrusion technique for depositing filaments according to the present invention. Filaments are deposited by coextruding different suspensions P1, P2 through separated inner parts 3 and outer parts 2 of the nozzle 1, thereby forming filaments showing an internal zone (with diameter D1) and an outer sheath (with thickness D2, whereby D2={(total diameter of the filament - D1)/2}. For instance, filaments can be deposited having an internal zone of diameter D1 made of suspension B or C and a sheath of thickness D2 made of suspension A. **FIG. 7B** is a cross sectional view of a filament that is obtained by means of the afore-mentioned co-extrusion technique and that shows an internal zone with diameter D1 and an outer sheath with thickness D2. As can be seen from **FIG. 7B**, microporosity of the internal zone and the outer sheath of the filament are different.

Suitable values for diameter D1 and thickness D2 depend on factors such as size and operation of the nozzles, composition of the suspension, etc. and can be readily determined by a skilled person. In a preferred embodiment the ratio of D2 to D1 is comprised between 0.3 and 2, and preferably between 0.5 and 1.

Using a co-extrusion technique, the present invention permits to prepare constructs having filaments showing a different distribution (degree) of micropores in the inner core than in the outer sheath of the filaments, in particular a higher microporosity in the outer sheath than in the internal zone. This is in contrast to filaments that have for instance been obtained after partial sintering, i.e. filaments that have been obtained when applying a sintering step as defined herein, but at lower temperatures and/or for shorter periods of time as those defined herein for the sintering step. In the latter case, partially sintered filaments have micropores that are typically uniformly distributed over the whole filaments and there are no significant differences in distribution of micropores between the internal zone and the outer sheath of the filaments, thus there are not more micropores in the sheath than in the internal zone.

In yet another embodiment, the present invention may also include the co-extrusion of a suspension as defined herein using profiled nozzles. Nozzles or nozzle heads for use in co-extrusion techniques are typically characterised by a body 1, which can be connected to a nozzle tube. The body 1 has an outer wall 12 and an inner wall 13 dividing the nozzle into outer parts 2 and an internal zone 3. The inner surface 11 of the outer wall of such nozzle may be profiled, as defined herein. Filaments obtained using this type of profiled nozzles also includes the advantages of combining surface roughness and microporosity and geometric roughness as explained above.

The present method includes the production of a 3D structure or scaffold by controlled deposition of e.g. extruded filaments in a non-solvent environment following a planned pattern. The necessary solidification of the filament is achieved by optimization of the air gap between the nozzle tip, the deposition layer, the non-solvent environment, the temperature and the air flow over the sample (vapour induced phase inversion - see below), and/or the temperature and composition of the immersion bath (immersion induced phase inversion - see below). Depending on the viscosity of the paste (viscosity between 100 - 10000 Pa s at a shear rate of 1 s⁻¹ and a yield point between 100 or 1000 Pa) several deposition parameters are optimized including:
■ the "air gap", meaning the distance between the nozzle and the surface of the structure. Typically this air gap is e.g. between 0 and 5 mm, and e.g. around 2 mm.
■ the non-solvent environment creating initial vapour induced phase inversion,
■ the speed of the nozzle movement between 0.5 and 5 m/min and e.g. around 2m/min, and
■ the air pressure of between 1 to 10 bar,
■ the temperature of the environment preferably of between 10 and 45°C,
■ the air flow over the sample during deposition,
■ the composition and the temperature of the non-solvent bath when immersing.

By carefully tuning these parameters, deposition of filaments without deformation and with a good contact between the layers can be obtained.

In a preferred embodiment, the method deposition of filaments of the suspension in a predetermined pattern at room temperature. In a preferred embodiment, the predetermined pattern can be a 0/90 (90 degree) pattern, i.e. forming parallel rows of filaments that cross each other perpendicularly, in a 0/45 (45 degree) pattern, i.e. forming parallel rows of filaments that cross each other diagonally, in a staggered (60 degree) pattern, i.e. forming parallel rows of filaments that cross each other an angle of 60°, in a cross-like pattern, or the like.

In another preferred embodiment, the method provides for the deposition of the suspension in filaments, whereby said predetermined pattern results in a trabecular lattice structure.

Practically, the paste can be placed in the dispending unit consisting of a syringe vessel and a nozzle. The unit can be mounted on a CNC machine and connected with an air pressure plunger to regulate the flow of the paste. For co-extrusion a system with a double cylinder syringe vessel and double cylinder nozzle can be used (see e.g. FIG. 6A). Deposition of the paste filaments can be controlled by the software program of the CNC machine. Depending on the viscosity of the paste, the deposition parameters, e.g. distance of the nozzle, specimen surface, air pressure, the speed of the movement of the nozzle, etc... can be regulated.

In a further embodiment, the method comprises de-airing of the paste in order to exclude air bubbles formation in the paste causing large defects (macrovoids) in the final structure or construct.

### STEP c)

A next step in the present method comprises the phase inversion process by bringing the filaments in contact with non-solvent. Preferably, phase inversion is induced by exposing the filaments during the deposition of the filaments to a non-solvent vapour and to a liquid non-solvent. In a preferred embodiment, during deposition, non-solvent vapour is blown over the filaments (step c1). When completed, the structure is immersed in a liquid non-solvent (step c2) whereby further phase-inversion is induced, thereby creating a filament-based porous structure having suitable filament morphology.

Vapour Induced Phase inversion is performed by creating a non-solvent environment and by flowing a suitable non-solvent vapour over the filaments during deposition of the filaments (step c1).

Immersion Induced Phase inversion and thus creation of the microporous filament structure with specific filament surface morphology is performed by dipping green structures in a liquid non-solvent for the phase inversion binder (polymer) (step c2). "Green" structures refer to structures that have not undergone a thermal treatment. Suitable liquid non-solvents include water, an alcohol, an acid, or a mixture thereof. Suitable examples of alcohol comprise but are not limited to methanol, ethanol, n-propanol, iso-propanol, n-butanol, octanol. Suitable examples of acids comprise but are not limited to acetic acid. A non-solvent/solvent mixture can also be used in this step. To optimize the morphology of the structure, addition of a (liquid) solvent as defined herein to the non-solvent can change the morphology structure of the filaments.

To create microporosity and surface roughness in the filaments a suspension composition as defined above is used which can undergo phase inversion. Normal sinter conditions can be performed resulting in structures with a controllable degree of microporosity and pore size distribution, the possibility of creating gradient porosity within the filaments and a higher strength than in the case of partial sintering, the conventional way of producing microporous structures.

Because microporosity is created when solvent is replaced by non-solvent and by pyrolysis of the polymer (binder) the microporosity is intrinsically interconnected. During sintering, shrinkage of the construct occurs, thereby lowering the total porosity and pore diameter.

Parameters optimizing this phase inversion and thus tailoring the filament structure are the composition of the suspension, extrusion parameters, rate of dipping, residence time in the non-solvent, type of non-solvent, addition of solvent to non-solvent and temperature.

### STEP d)

In a further step in the present method the structure of step c) is thermally treated by calcining and sintering said structure.

Preferably, the structure of step c) is dried prior to being calcined and sintered in vacuum. Drying may for instance be done at room temperature by air drying or in a controlled atmosphere (temperature, humidity).

The method preferably comprises a calcination step which comprises a heating step wherein the green structure obtained after step c) of the method is heated at a rate between 5 to 50°C/hour to a temperature between 200 and 600°C, and for instance between 400 and 600°C, and preferably below 500°C. Preferably, especially in the case of the use of Ti or Ti-alloy powder particles in the suspension, calcination is performed under an inert atmosphere such as argon or at low pressure, preferably at a pressure of less than 10⁻³ mbar. During this step most of the organic material will be pyrolysed.

The present method further comprises a pre-sintering step between the calcination step and the sintering step, comprising heating up the construct until a temperature of between 900 and 1000°C is reached. In the case of the use of Ti or Ti-alloy powder particles in the suspension, pre-sintering results in a structure where the Ti or Ti-alloy powder particles already start to sinter together, allowing handling of the structure. Preferably, this step is performed in an inert atmosphere or under a vacuum of at least 10⁻⁴ mbar on a Y₂O₃-coated substrate.

Next, and especially in the case of the use of Ti or Ti-alloy powder particles in the suspension, the obtained structure is sintered on a Y₂O₃- coated substrate or on a Y₂O₃ powder bed, allowing a further shrinking of the structure. Again, an inert atmosphere or a high vacuum of more than 10⁻⁴ mbar is used. Sintering is preferably carried out by heating at a heating rate of between 1 to 10°C /min to a temperature of between 1000 and 1500°C, and for instance of between 1200 and 1500°C and keeping said structure at said temperature for a pre-determined period of time of between 1 to 5 hours, where after a cooling to room temperature follows at a rate of e.g. 20°C/min.

Depending on the dimension requirements, a final machining step can be performed, e.g. drilling, cutting.

### B. Characteristics of the 3D macroporous filament construct

In accordance with the invention, a three-dimensional macroporous filament construct comprising filaments with interconnected micropores having a suitable morphology, is obtained.

Preferably, the invention provides a three-dimensional macroporous filament construct made of a predetermined material selected from the group comprising metal, ceramic or composite materials, and preferably Ti or a Ti alloy, and comprising filaments having an average surface roughness (Ra) which is higher than 4µm. In another preferred embodiment, the filaments have interconnected microporosity whereby said microporosity is between 1 and 50%, preferably between 5 and 30%.

More preferably, the invention provides a three-dimensional macroporous filament construct for adhering a bone promoting protein (BMP), stem cells, osteoblast cells, pharmaceuticals or/and a mixture thereof made of a predetermined material selected from the group comprising metal, ceramic or composite materials, and preferably Ti or a Ti alloy, comprising filaments having an average surface roughness (Ra) which is higher than 4µm, and having between 1 and 50%, preferably between 5 and 30% of interconnected micropores, which micropores have a pore size equal to or smaller than 100µm.

As indicated above, constructs wherein both of these parameters of surface roughness and microporosity are provided in a single construct have not been reported and are therefore unconventional and unusual.

In another preferred embodiment the invention provides a three-dimensional macroporous filament construct having a specific surface area of at least 1m²/g, and preferably of at least 1.5 m²/g

In a preferred embodiment said three-dimensional macroporous filament construct has a macroporosity of at least 50% and preferably in the range from 50 to 95 %, more preferably between 60 to 85 %. In other words, a construct is provided having between 50 and 95 %, preferably between 60 and 85 % of macropores, wherein said macropores consist of pores having a pore size greater than 100µm.

In another embodiment said three-dimensional macroporous filament construct has macropores having a pore size between 100 and 2000 µm, and for instance of between 200 and 1000µm.

In yet another embodiment said three-dimensional macroporous filament construct, and preferably when made from Ti or Ti-alloy powder particles in the suspension, has a compressive modulus (E-modulus) lower than 3 GPa, and preferably of between 2 and 3 GPa.

In another embodiment said three-dimensional macroporous filament construct, and preferably when made from Ti or Ti-alloy powder particles in the suspension, has a ductility of between 3 and 20 %, preferably of between 5 and 15%. Ductility is commonly defined as the ability of a material to deform easily upon the application of a tensile force, or as the ability of a material to withstand plastic deformation without rupture, breaking or fracturing. Ductility may also be thought of in terms of bend ability and crushability. Ductile materials show large deformation before fracture. The lack of ductility is often termed brittleness. Ductility can be measured using a compression test method which is well known in the prior art (see for instance the textbook "Metal Foams", Ashby et al., Ed. By Butterworth-Heinnemann, 2000).

In an example, the present method yields said porous structure which is a ductile Ti-(or Ti-alloy) highly porous (macroporosity of at least 50%) scaffold, that can be deformed more than 7 % in compression without rupture.

In another preferred embodiment, the three-dimensional macroporous filament construct , and preferably when made from Ti or Ti-alloy powder particles in the suspension, according to the invention has limited concentrations of contaminants, and for instance has an amount of O equal to or lower than 0.8wt%, an amount of N equal to or lower than 0.5wt%, an amount of C equal to or lower 0.03wt%, an amount of Fe equal to or lower 0.1wt%, and an amount of Si equal to or lower 0.1wt%.

The invention also relates to a construct wherein said filament surface is modified with a collagen or calciumphosphate coating.

In yet another embodiment, the invention provides a construct, comprising filaments showing an internal zone and an outer sheath, and wherein the amount of said micropores in said filaments is different in said internal zone and in said outer sheath. Preferably a construct comprising filaments showing an internal zone and an outer sheath is provided wherein the amount of said micropores is lower in said internal zone than in said outer sheath, especially for reason of mechanical strength of the filaments. Preferably a construct is provided wherein the filaments have between 1 and 50%, and preferably between 5 and 30% micropores in the outer sheath between 1 and 10%, and preferably between 1 and 5% micropores in the internal zone.

In addition, the invention may further provide a construct, comprising filaments showing an internal zone and an outer sheath, and wherein the amount of said micropores in said filaments is different in said internal zone and in said outer sheath, and wherein the filaments are profiled filaments are defined herein. Preferably a construct comprising profiled filaments, showing an internal zone and an outer sheath is provided wherein the amount of said micropores is tower in said internal zone than in said outer sheath, especially for reason of mechanical strength of the filaments. Preferably a construct is provided wherein the profiled filaments have between 1 and 50%, and preferably between 5 and 30% micropores in the outer sheath between 1 and 10%, and preferably between 1 and 5% micropores in the internal zone.

### C. Applications and uses

The present invention can be used for the production of highly porous constructs which can be applied as orthopaedic implant material and tissue engineering matrices. The highly porous structures having an adjustable pore size distribution and controllable microporosity within the struts make these materials suitable to optimize osteoinduction and osteoconduction.

In a preferred embodiment, the invention provides a synthetic bone material that is obtainable by a method according to the present invention.

In another preferred embodiment, the invention provides a composition which comprises a 3D macroporous filament construct as defined herein and further comprising a bone promoting protein (BMP), stem cells, osteoblast cells, pharmaceuticals or/and a mixture thereof. Non-limitative examples of suitable pharmaceuticals may include antibiotics such as e.g. gentamycin, vancomycin.

The invention further provides for the use of a construct or of a composition as defined herein for the manufacture of a biomedical product, such as but not limited to a synthetic bone implant, a synthetic bone material, a bone graft, a tissue engineering scaffold, a drug-delivery device. Biomedical products as referred to herein may include e.g. instruments, devices, implants and the like intended for use in the diagnosis, treatment, or prevention of a disease or other conditions which does not achieve its primary intended purpose through chemical action, and which is not dependent upon being metabolized for the achievement of its primary intended purpose.

The invention thus also provides a biomedical product such as a synthetic bone implant or bone graft, a tissue engineering scaffold, a drug-delivery device comprising a construct or a composition as defined herein. For instance, in accordance with the invention and by careful control and selection of the starting materials and the process parameters a regular or irregular Ti or Ti alloy porous structure is obtained, that is highly ductile and with micro and macro porosity and morphology parameters suitable as orthopaedic material and more specific synthetic bone material for use in biomedical applications. When used as bone implant, bone graft, or tissue engineering scaffold, the present construct or composition is able to perform as a substrate that will support the appropriate cellular activity, including the facilitation of molecular and mechanical signalling systems, in order to optimize tissue regeneration, without eliciting any undesirable effects in those cells, or inducing any undesirable local or systemic responses in a patient.

In addition, the use of a 3D macroporous filament construct or composition as defined herein is not limited to (bio)medical application. Other applications for instance non-medical applications of the present constructs are also possible. For instance, the present 3D macroporous filament construct as defined herein can be used as catalyst support, filter, lightweight structure, mechanical damping device, sensor, electrode, heat exchanger or charge carrier.

The present invention is further illustrated by means of the following examples and drawings.

### Examples

### Example 1: Method according to the invention

The present invention is related to a method for producing a three-dimensional macroporous filament structure with interconnected microporous filaments and rough surface topography.

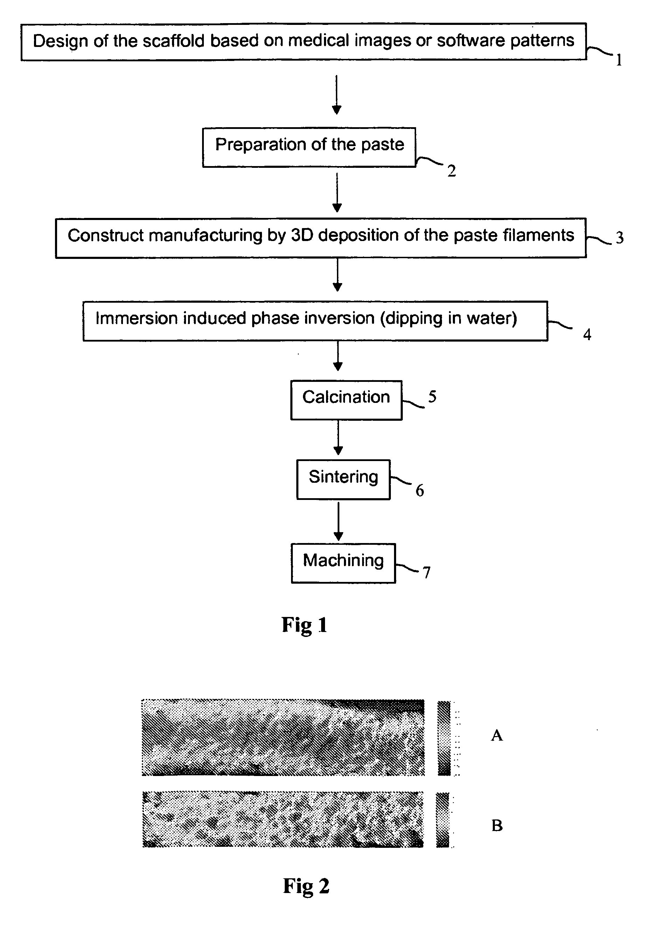

It comprises the following steps, as illustrated in **FIG. 1**:
- based on medical images or predefined software patterns, a near net shape design of the scaffold is made in box 1, taking into account the shrinkage of the scaffold during the sintering. Eventually some over-dimensioning or simplification of the shape can be foreseen, allowing the use of final machining in order to obtain the required dimensions and tolerances;
- than a powder paste is prepared in box 2 by e.g. mixing Ti- (or Ti-alloy) powder with a 20 to 85 weight % and a mixture of binder, plasticizers and dispersants in a solvent. The binders are a mixture of a phase-inversion binder and a rheology modification binder. The paste is brought in a dispending unit consisting of a syringe vessel and a nozzle. The unit is mounted on a CNC machine and connected with an air pressure plunger to regulate the flow of the paste. Co-extrusion can be performed with an adapted system;
- the CNC-machine is programmed to move according to a well defined pattern and within a well defined form. The CNC machine is programmed to continuously deposit the filaments layer by layer in a predefined pattern. Depending on the thickness of the filaments foreseen in the application a nozzle with the right diaphragm opening will be chosen e.g. between 0.1 and 2 mm. The deposition parameters, e.g. the distance between the nozzle and the surface of the structure, non-solvent environment for vapour induced phase inversion to solidify the filaments, the speed of the nozzle movement, the air pressure and the temperature and airflow of the environment, etc.. are regulated. A 3D-structure is built in box 3 by depositing the filaments layer by layer according to the programmed pattern and according to the required dimensions;
- microporosity of the filament struts is created when bringing the filaments during deposition in contact with the non-solvent environment (box 4) whereafter the scaffold is dried;
- then the structure undergoes thermal treatment by calcination under inert atmosphere or vacuum in box 5 and sintering under inert atmosphere or vacuum in box 6;
- depending of dimension requirements a final machining step can be performed in box 7.

### Example 2: Effect of the amount of a PSf binder in a suspension of the invention

The following example shows the influence of the amount of a phase inversion binder (PSf) used in a suspension according to the invention on microstructural and mechanical properties of constructs according to the invention, including surface roughness as measured on the surface of a single filament; microporosity in the filaments; specific surface area (SSA); and ductility. The experiments are compared with a reference measurement, performed on a sample which is fabricated without any phase binder added in the suspension.

A construct was made in accordance with the present method based on a suspension comprising Ti powder (55 - 95 wt %), ethyl cellulose (0.1 - 3 wt %), N-methyl pyrrolidone (5 - 30 wt %), dibutyl phtalate (1.4 wt%), and 0.1 - 1 wt % of a wax. The amount of the phase inversion binder was varied between 0.5 - 20 wt % (on Ti basis).

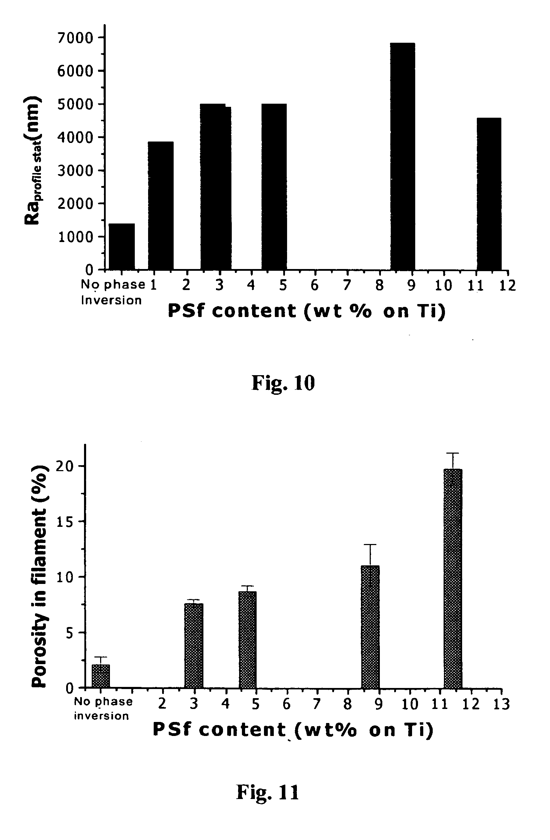

**Fig. 10** illustrates the influence of the amount of a polysulphone phase inversion binder (in wt% PSf) on the surface roughness (Ra) of filaments in a construct according to the invention. Surface roughness is measured by non-contact, optical profilometry based on interferometry (VEECO, Wyko NT3300 - A.G. Olszak, J. Schmit, M.G. Heaton, "Interferometry: Technology and Applications," Veeco Instruments, Inc., 2650 E. Elvira Road, Tucson, AZ 85706, 2001).

**FIG. 10** presents the trend in roughness measured on the surface of a single filament, when increasing amounts of PSf are present in the suspension that is deposited by this method. The values are compared to the roughness of a filament deposited from a paste without any phase inversion binders present. The experiment showed that the induction of microporosity in the filament by the present invention leads to a roughened surface of the filament, even after addition of low amount of PSf binder in the suspension.

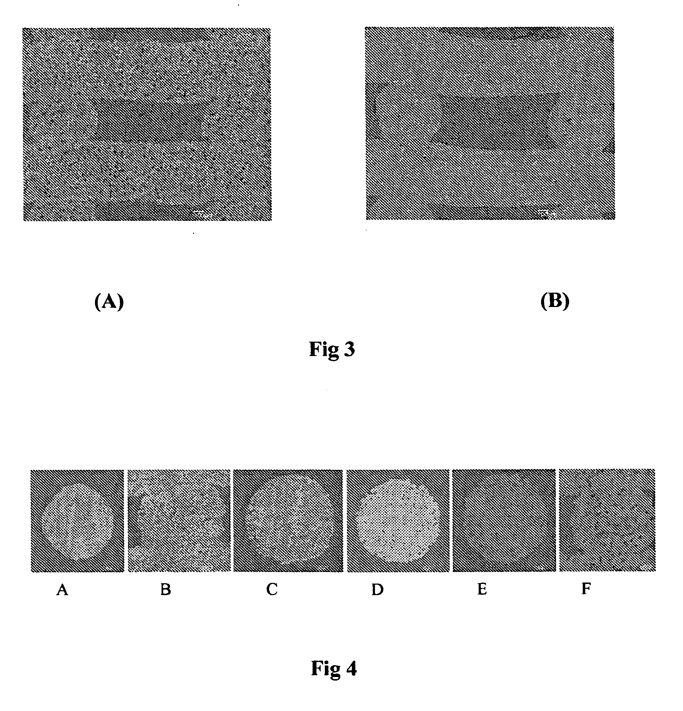

Porosity within the filament (microporosity) is measured using image analysis on SEM pictures. Based on grey value differences, the software (KS 400, C. Zeiss) is able to count the ratio of pixels representing porosity and pixels representing material. **FIG. 11** illustrates the influence of the amount of a polysulphone phase inversion binder (in wt% PSf) on microporosity (in %) of filaments in a construct according to the invention. Increasing the amount of phase inversion binder in the suspension that is to be deposited causes an increase in microporosity within the filaments (see **FIG. 11**). The same trend is observed in **FIG. 4** which shows SEM images of cross-sections of filaments obtained with variation in % polysulphone binder (wt% polysulphone PSf on Ti) from 0.00% (A); 2.78 % (B), 2.96 %(C), 4.72% (D), 8.7% (E) and 11.39% (F).

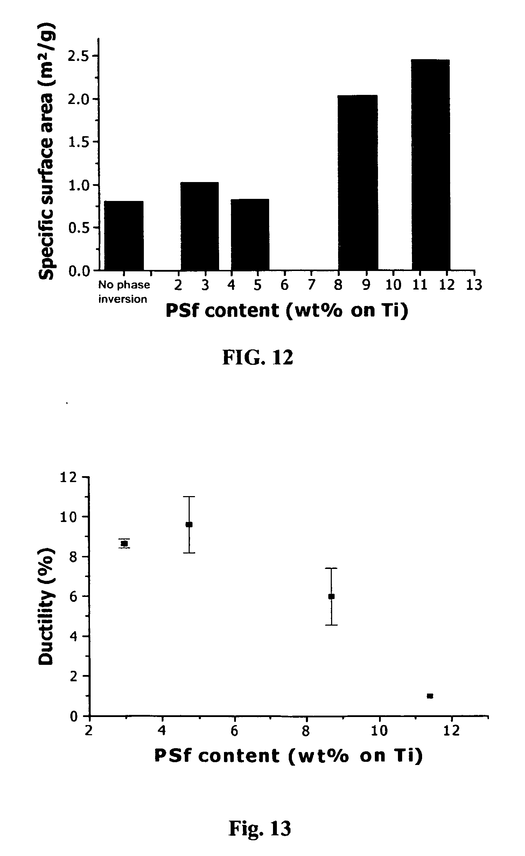

Specific surface area is measured by gas adsorption (Quantochrome, N₂ adsorption). **FIG. 12** illustrates the influence of the amount of a polysulphone phase inversion binder (in wt% PSf) on the specific surface area (SSA in m²/g) of a construct according to the invention. **FIG. 12** presents the evolution of the specific surface area of sintered structures with increasing amount of phase inversion binder in the suspension used to form the constructs. It is illustrated that inducing the microporosity within the filaments leads to an increased surface area of the filaments and as such of the construct.

Ductility is the degree of plastic deformation of a structure without rupture, when compressed. **FIG. 13** illustrates the influence of the amount of a polysulphone (in % PSf) on ductility (in %) of filaments in a construct according to the invention. **FIG. 13** presents the evolution of the ductility of the constructs formed by the present invention with increasing amount phase inversion binder (PSf) in the suspension. Ductility of a macroporous structure according to the invention can be controlled in function of type and amount of phase inversion binder applied in a suspension according to the invention.

### Example 3: Effect of a cellulose acetate binder in a suspension of the invention

Example 3 of this invention comprises the use of an alternative phase inversion binder. Here, the composition of the suspension to be extruded is based on cellulose acetate (CA) as phase inversion binder. As solvent for CA, di-methyl formamide (DMF) was selected. The suspension comprises Ti-powder (80 wt%), cellulose acetate (4.8 wt%), DMF (13 wt%), ethyl cellulose (0.15 wt%) and a wax (1.5 wt%).

The suspension was placed into dispensing unit and the complete procedure was followed as described in example 1 of the present invention. Again, water was selected as non-solvent.

After sintering, the resulting microporosity within the filaments is noticed in the SEM-picture of the cross section (FIG. 5). Image analysis on the SEM-picture of the cross section revealed a porosity within the filament of 7.9 (± 0.2) %.

Table 1 illustrates the influence of the application of a phase inversion technique in the method of preparing a construct according to the invention.

**Table 1**

| **Characteristic** | **Analytical Tool** | **Influence of phase inversion¹** | **Relevance / requirement** |
|---|---|---|---|
| Total Porosity | Geometric | + | Induced microporosity increases total porosity as porosity is created within the filaments of the construct. |
| Microporosity | Hg-intrusion porosimetry | + | Interconnected microporosity within the filaments improves cell attachment, promotes cell behaviour within the construct (differentiation to bone cells) and enables nutrient transport within the filaments of the constructs. Facilitates additional coatings on filaments of the construct. |
| Specific surface area | N₂-adsorption | + | Increased specific surface area augments cell attachment sites or sites for binding other biological active substances. Promotes cell behaviour within the construct and enables nutrient transport within the filaments of the constructs. |
| Compression strength | Compression test | - | Induced microporosity lowers the mechanical strength of construct, optimizing bone sinking and preventing stress shielding effects |
| E modulus | Compression test Impulse excitation test | - | Lowered E-modulus prevents stress shielding effects |
| Coefficient of friction (COF) | Friction test | + | Increased COF minimizes the micromotion of implants with surrounding tissue, and as such enables and enhances bone formation and bone ingrowth |
| Fatigue strength | Fatigue test | - | Induced microporosity has negative influence on fatigue strength (5 million cycles applying a load of 25 MPa is regarded as the minimum standard). |
| Wicking speed | Wicking speed measurement | + | Induced microporosity within the filaments allows nutrients to flow not only through the macropores, but offer alternative and faster routes for fluid transport within the filaments (capillarity), resulting in optimized nutrient transport. |
| Strut size | SEM-IA² µ-CT³ | ni | None, present invention has no influence on this parameter |
| Pore size | SEM-IA µ-CT | ni | None, present invention has no influence on this parameter |
| Filament surface roughness | Optical profilometry | + | Increased surface roughness leads to higher coefficient of friction, optimized bone cell attachment and differentiation and facilitates additional coatings on filaments of the construct |
| Chemical composition | Elemental analysis | Dependent on phase inversion binder | Ideally, phase inversion binders are to be selected to minimize the residual contaminants after sintering, because of their detrimental effect on mechanical strength |
| Ductility | Compression test | Dependent on phase inversion binder | Ideally, phase inversion binders are to be selected to minimize the residual contaminants after sintering, because of their detrimental effect on the ductility |
| Filament pattern | SEM-IA | ni | None, present invention has no influence on this parameter |

| | | | |
|---|---|---|---|
| ¹ referenced to samples manufactured without phase inversion binders; "+" = increase, "-" = decrease; "ni"= no influence ² Scanning electron microscopy - image analysis ³ Micro-computerized tomography | | | |

The methods and techniques for measuring the above-indicated characteristics are well-known in the present field of material technology and are therefore not described in detail herein.

### Example 4: Co-extrusion with controlled microporous filament sheath.

Example 4 is directed to the extrusion of two compositionally different Ti-6Al-4V (Ti-alloy) based pastes through a specifically designed nozzle (as illustrated on **FIG. 6A**). As illustrated on **FIG. 6A**, a nozzle is composed of two concentric apertures, each fed by a separate syringe, allowing the co-extrusion of two pastes. This set up results in a filament which is composed of an inner part (with diameter D₁) and an outer layer (with a thickness D₂). As the composition of both pastes is different, a different microstructure can be obtained in the inner part of the filament compared to the outer layer of the filament. Changing the nozzle openings allows the variation of D₁ and/or D₂.

In example 4, the inner part of the filament originates from a paste without phase inversion binder, while the outer layer of the filament originates from a paste which contains phase inversion binder. Consequently, only the outer layer of the filament will undergo phase inversion, resulting locally in the formation of a high degree of microporosity. The inner part of the filament can not undergo phase inversion, resulting in a very low degree of microporosity.

The paste for the inner part of the filament contains Ti-6Al-4V powder (87 wt%), methyl cellulose (0.4 wt%), dibutyl phtalate (1.4 wt%), a wax (0.5 wt%) and water (10.5 wt%). The paste for the outer layer of the filament contains Ti-6Al-4V powder (82 wt%), methyl cellulose (1 wt%), polysulphone (2 wt%) and N-methyl pyrrolidone (15 wt%).The pressure used for the extrusion of the paste in the inner part of the nozzle was 6 bar (P₁ in FIG. 6) and was 3 bar for the extrusion of the paste in the outer part of the nozzle (P₂ in FIG. 6).

During deposition, an atmosphere with high humidity was blown over the structure, in order to start the phase inversion process in the outer layer of the filament. After depositing the complete structure, the sample was submerged in liquid water to complete the phase inversion process in the outer layer of the filament.

After drying, the sample was calcined and sintered.

The microstructure of the sample was investigated using a light microscope. **FIG. 7A** shows both the longitudinal sections and cross sections of the filaments, revealing the high degree of microporosity (black spots), being substantially only present in the outer layers of the filaments. The inner part of the filaments is characterised by a low degree of porosity, substantially originating from residual entrapped air in the paste. In the present example, the porosity in the outer layer of the filament is 14.4 % (**FIG. 7B** - area D₂), while the porosity in the inner part of the filament is only 1.8 % (**FIG. 7B** - area D₁).

In conclusion, the present invention provides a macroporous construct produced by a novel and innovative method. The present constructs are particularly suitable for use as implant and provide maximum bone cell differentiation. The macroporous construct is characterized by fully interconnected inter- and intra-filamentary porosity, controlled filament morphology, a suitable microporosity for rapid bone ingrowth, homogenously distributed micropores and high ductility. Moreover, the present constructs are defect free and have optimized filament surface. The present method is characterized by a direct suspension extrusion into filaments and can be carried out completely at room temperature. It can be applied to a.o. metal and ceramic materials and a dual co-axial microporosity capability (e.g. different microporosity in inner core and outer sheath of filaments) can be obtained.

## Claims

1. A method for producing a three-dimensional macroporous filament construct having interconnected microporous filaments and a suitable morphology, said method comprising the steps of:
a) preparing a suspension comprising particles of a predetermined material, a liquid solvent, one or more binders and optionally one or more dispersants,
b) depositing said suspension in the form of filaments in a predetermined three-dimensional pattern thereby creating a three-dimensional filament-based porous structure,
c) inducing phase inversion whereby said filaments are transformed from a liquid to a solid state by the steps of
c1) bringing said filaments during the deposition of the filaments into contact with a non-solvent vapour, and
c2) immersing the structure of step c1) in a liquid non-solvent, thereby creating a filament-based porous structure having suitable filament morphology,
d) thermally treating the structure of step c) by calcining and sintering said structure.

2. Method according to claim 1, wherein step b) is carried out in a non-solvent environment.

3. Method according to claim 1 or 2, wherein the filaments in the filament-based porous structure comprise an average surface roughness (Ra) which is higher than 4µm.

4. Method according to any of claims 1-3, wherein the filaments in the filament-based porous structure have between 1 and 50%, preferably between 5 and 30% of micropores, wherein said micropores consist of pores having a pore size equal to or smaller than 100µm.

5. Method according to any of claims 1-4, wherein said predetermined material is selected from the group comprising metal, ceramic or composite materials, and preferably is Ti or a Ti alloy.

6. Method according to any of claims 2-5, wherein said non-solvent environment is an environment with a relative non-solvent vapour of at least 10%.

7. Method according to any of claims 2-6, wherein said non-solvent environment is a humidifying environment or ambient air with a relative humidity of at least 50%.

8. Method according to any of claims 2-7, wherein said non-solvent environment is created by a gas stream of water vapour.

9. Method according to any of claims 1-8 wherein deposition of said suspension in the form of filaments is performed by extrusion of said suspension through a nozzle having a profiled inner surface.

10. Method according to any of claims 1-9, wherein deposition of filaments is performed by co-extrusion of different suspensions (P1, P2) through separated inner parts (3) and outer parts (2) of a nozzle (1), thereby forming filaments showing an internal zone and an outer sheath.

11. Method according to claim 10, wherein the amount of said micropores in said filaments is different in said internal zone and in said outer sheath.

12. Method according to any of claims 1-11, wherein said liquid non-solvent is water, an alcohol or an acid.

13. Method according to any of claims 1-12, wherein calcining comprises heating the structure of step c) to a temperature comprised between 400 and 600°C and at a rate between 5-50°C/hour.

14. Method according to any of claims 1-13, wherein sintering comprises
- heating said structure to a temperature comprised between 1200 and 1500°C and at a rate between 1-10°C/minute,
- keeping said structure at said temperature for a period of time between 1 to 5 hours, and
- subsequently cooling said structure at a rate of 20°C/minute to room temperature.

15. Three-dimensional macroporous filament construct obtainable by the method according to any of claims 1 to 14, comprising filaments which have between 1 and 50%, preferably between 5 and 30% of interconnected micropores, wherein said micropores consist of pores having a pore size equal to or smaller than 100µm, and which filaments have an average surface roughness (Ra) higher than 4µm.

16. Construct according to claim 15 wherein said macroporous filament construct has between 50 and 95 %, preferably between 60 and 85 % of macropores, wherein said macropores consist of pores having a pore size greater than 100µm.

17. Construct according to any of claim 15 or 16, wherein said macroporous filament construct has a compressive modulus lower than 3 GPa.

18. Construct according to any of claims 15-17, wherein said macroporous filament construct has a ductility of between 3% and 10%, preferably of between 5% and 15%.

19. Construct according to any of claims 15-18, comprising filaments showing an internal zone and an outer sheath, and wherein the amount of said micropores in said filaments is different in said internal zone and in said outer sheath, and preferably lower in said internal zone than in said outer sheath.

20. A composition which comprises a construct as claimed in any of claims 15-19 and further comprising a bone promoting protein (BMP), stem cells, osteoblast cells, pharmaceuticals or/and a mixture thereof.

21. Use of a construct as claimed in any of claims 15-19 or a composition as claimed in claim 20 for the manufacture of a (bio)medical product, such as a synthetic bone implant or bone graft, a tissue engineering scaffold, a drug-delivery device.

22. Biomedical product such as a synthetic bone implant or bone graft, a tissue engineering scaffold, a drug-delivery device comprising a construct as claimed in any of claims 15-19 or a composition as claimed in claim 20.

## Patentansprüche

1. Verfahren zur Herstellung eines dreidimensionalen makroporösen Filamentkonstrukts, das miteinander verbundene mikroporöse Filamente und eine geeignete Morphologie aufweist, wobei das Verfahren folgende Schritte umfasst:
a) Herstellen einer Suspension, die Partikel eines vorab festgelegten Materials, ein flüssiges Lösemittel, ein oder mehrere Bindemittel und optional ein oder mehrere Dispergiermittel umfasst,
b) Ablagern der Suspension in Form von Filamenten in einem vorab festgelegten dreidimensionalen Muster, wodurch eine dreidimensionale filamentbasierte poröse Struktur erzeugt wird,
c) Herbeiführen einer Phasenumkehr, wobei die Filamente durch folgende Schritte von einem flüssigen Zustand in einen festen Zustand überführt werden:
c1) In-Kontakt-Bringen der Filamente mit einem Nichtlösemitteldampf und
c2) Tauchen der Struktur aus Schritt c1) in ein flüssiges Nichtlösemittel, wodurch eine filamentbasierte poröse Struktur mit geeigneter Filamentmorphologie erzeugt wird, und
d) Wärmebehandeln der Struktur aus Schritt c) durch Kalzinieren und Sintern der Struktur.

2. Verfahren nach Anspruch 1, wobei Schritt b) in einer Nichtlösmittelumgebung ausgeführt wird.

3. Verfahren nach Anspruch 1 oder 2, wobei die Filamente in der filamentbasierten porösen Struktur eine durchschnittliche Oberflächenrauheit (Ra) umfassen, die höher als 4 µm ist.

4. Verfahren nach einem der Ansprüche 1 bis 3, wobei die Filamente in der filamentbasierten porösen Struktur zwischen 1 und 50 %, vorzugsweise zwischen 5 und 30 % Mikroporen aufweisen, wobei die Mikroporen aus Poren bestehen, die eine Porengröße kleiner als oder gleich 100 µm aufweisen.

5. Verfahren nach einem der Ansprüche 1 bis 4, wobei das vorab festgelegte Material aus der Gruppe ausgewählt ist, die Metall, Keramik oder Verbundmaterialien umfasst, und vorzugsweise Ti oder eine Ti-Legierung ist.

6. Verfahren nach einem der Ansprüche 2 bis 5, wobei die Nichtlösemittelumgebung eine Umgebung mit einem relativen Gehalt an Nichtlösemitteldampf von mindestens 10 % ist.

7. Verfahren nach einem der Ansprüche 2 bis 6, wobei die Nichtlösemittelumgebung eine befeuchtende Umgebung oder Umgebungsluft mit einer relativen Luftfeuchte von mindestens 50 % ist.

8. Verfahren nach einem der Ansprüche 2 bis 7, wobei die Nichtlösemittelumgebung durch einen Gasstrom aus Wasserdampf erzeugt wird.

9. Verfahren nach einem der Ansprüche 1 bis 8, wobei das Ablagern der Suspension in Form von Filamenten durch Extrudieren der Suspension durch eine Düse mit profilierter Innenfläche ausgeführt wird.

10. Verfahren nach einem der Ansprüche 1 bis 9, wobei das Ablagern der Filamente durch Koextrusion verschiedener Suspensionen (P1, P2) durch getrennte Innenteile (3) und Außenteile (2) einer Düse (1) ausgeführt wird, wodurch Filamente gebildet werden, die einen Innenbereich und ein Außenhülle aufweisen.

11. Verfahren nach Anspruch 10, wobei sich in den Filamenten die Menge der Mikroporen im Innenbereich von der in der Außenhülle unterscheidet.

12. Verfahren nach einem der Ansprüche 1 bis 11, wobei das Nichtlösemittel Wasser, ein Alkohol oder eine Säure ist.

13. Verfahren nach einem der Ansprüche 1 bis 12, wobei das Kalzinieren das Erwärmen der Struktur aus Schritt c) auf eine Temperatur umfasst, die zwischen 400 und 600 °C liegt, mit einer Rate von 5 bis 50 °C/Stunde.

14. Verfahren nach einem der Ansprüche 1 bis 13, wobei das Sintern Folgendes umfasst:
- Erwärmen der Struktur auf eine Temperatur, die zwischen 1200 und 1500 °C liegt, mit einer Rate von 1 bis 10 °C/Minute,
- Halten der Struktur auf der Temperatur über einen Zeitraum zwischen 1 und 5 Stunden und
- anschließendes Kühlen der Struktur auf Raumtemperatur mit einer Rate von 20 °C/Minute.

15. Dreidimensionales makroporöses Filamentkonstrukt, das mit dem Verfahren nach einem der Ansprüche 1 bis 14 gewonnen werden kann, Filamente umfassend, die zwischen 1 und 50 %, vorzugsweise zwischen 5 und 30 % miteinander verbundener Mikroporen aufweisen, wobei die Mikroporen aus Poren bestehen, die eine Porengröße kleiner als oder gleich 100 µm aufweisen, und wobei die Filamente eine durchschnittliche Oberflächenrauheit (Ra) größer als 4 µm aufweisen.

16. Konstrukt nach Anspruch 15, wobei das makroporöse Filamentkonstrukt zwischen 50 und 95 %, vorzugsweise zwischen 60 und 85 % Makroporen aufweist, wobei die Makroporen aus Poren bestehen, die eine Porengröße größer als 100 µm aufweisen.

17. Konstrukt nach Anspruch 15 oder 16, wobei das makroporöse Filamentkonstrukt einen Kompressionsmodul kleiner als 3 GPa aufweist.

18. Konstrukt nach einem der Ansprüche 15 bis 17, wobei das makroporöse Filamentkonstrukt eine Duktilität zwischen 3 % und 10 %, vorzugsweise zwischen 5 % und 15 % aufweist.

19. Konstrukt nach einem der Ansprüche 15 bis 18, Filamente umfassend, die einen Innenbereich und eine Außenhülle aufweisen, und wobei sich in den Filamenten die Menge der Mikroporen im Innenbereich von der in der Außenhülle unterscheidet und vorzugsweise im Innenbereich kleiner als in der Außenhülle ist.

20. Zusammensetzung, die ein Konstrukt nach einem der Ansprüche 15 bis 19 umfasst und ferner ein das Knochenwachstum förderndes Protein (BMP), Stammzellen, Osteoblasten, Pharmaka oder/und eine Mischung daraus umfasst.

21. Verwendung eines Konstrukts nach einem der Ansprüche 15 bis 19 oder einer Zusammensetzung nach Anspruch 20 für die Herstellung eines (bio)medizinischen Produkts, wie beispielsweise eines synthetischen Knochenimplantats oder eines synthetischen Knochenersatzes, eines Gerüsts zur Gewebezüchtung (Tissue Engineering), einer Wirkstoffabgabeeinrichtung.

22. Biomedizinisches Produkt, wie beispielsweise ein synthetisches Knochenimplantat oder ein synthetischer Knochenersatz, ein Gerüst zur Gewebezüchtung (Tissue Engineering), eine Wirkstoffabgabeeinrichtung, das ein Konstrukt nach einem der Ansprüche 15 bis 19 oder eine Zusammensetzung nach Anspruch 20 umfasst.

## Revendications

1. Procédé de production d'une construction filamentaire et macroporeuse en trois dimensions, ayant des filaments microporeux interconnectés et une morphologie adaptée, ledit procédé comprenant les étapes consistant à :
a) préparer une suspension comprenant des particules d'une substance prédéterminée, un solvant liquide, un ou plusieurs liants et facultativement un ou plusieurs dispersants,
b) déposer ladite suspension sous la forme de filaments selon un profil en trois dimensions prédéterminé, créant ainsi une structure poreuse à base de filaments en trois dimensions,
c) induire une inversion de phases, lesdits filaments étant ainsi transformés d'un état solide à un état liquide par les étapes consistant à :
c1) mettre lesdits filaments en contact avec une vapeur sans solvant durant le dépôt des filaments, et
c2) immerger la structure de l'étape c1) dans un non solvant liquide, créant ainsi une structure poreuse à base de filaments ayant une morphologie filamentaire adaptée,
d) traiter thermiquement la structure de l'étape c) par calcination et frittage de ladite structure.

2. Procédé selon la revendication 1, l'étape b) étant réalisée dans un environnement sans solvant.

3. Procédé selon la revendication 1 ou 2, les filaments dans la structure poreuse à base de filaments ayant une rugosité moyenne de surface (Ra) qui est supérieure à 4 µm.

4. Procédé selon l'une quelconque des revendications 1 à 3, les filaments dans la structure poreuse à base de filaments ayant de 1 à 50 %, de préférence de 5 à 30 % de micropores, lesdits micropores étant constitués par des pores ayant une taille de pores inférieure ou égale à 100 µm.

5. Procédé selon l'une quelconque des revendications 1 à 4, ledit matériau prédéterminé étant choisi dans le groupe comprenant les matériaux métalliques, céramiques ou composites, et étant de préférence le Ti ou un alliage de Ti.

6. Procédé selon l'une quelconque des revendications 2 à 5, ledit environnement sans solvant étant un environnement avec une vapeur sans solvant relative d'au moins 10 %.

7. Procédé selon l'une quelconque des revendications 2 à 6, ledit environnement sans solvant étant un environnement humidifiant ou l'air ambiant ayant une humidité relative d'au moins 50 %.

8. Procédé selon l'une quelconque des revendications 2 à 7, ledit environnement sans solvant étant créé par un courant gazeux de vapeur d'eau.

9. Procédé selon l'une quelconque des revendications 1 à 8, le dépôt de ladite suspension sous la forme de filaments étant réalisé par extrusion de ladite suspension par une buse ayant une surface intérieure profilée.

10. Procédé selon l'une quelconque des revendications 1 à 9, le dépôt de filaments étant réalisé par coextrusion de différentes suspensions (P1, P2) par des parties intérieures (3) et des parties extérieures (2) d'une buse (1) séparées, formant ainsi des filaments présentant une zone interne et une gaine externe.

11. Procédé selon la revendication 10, la quantité desdits micropores dans lesdits filaments étant différente dans ladite zone interne et dans ladite gaine externe.

12. Procédé selon l'une quelconque des revendications 1 à 11, ledit non solvant liquide étant l'eau, un alcool ou un acide.

13. Procédé selon l'une quelconque des revendications 1 à 12, la calcination comprenant le chauffage de la structure de l'étape c) à une température comprise entre 400 et 600°C et à une vitesse comprise entre 5 et 50°C/heure.

14. Procédé selon l'une quelconque des revendications 1 à 13, ledit frittage comprenant :
- le chauffage de ladite structure à une température comprise entre 1200 et 1500°C et à une vitesse comprise entre 1 et 10°C/minute,
- le maintien de ladite structure à ladite température pendant une durée comprise entre 1 et 5 heures, et
- le refroidissement subséquent de ladite structure à une vitesse de 20°C/minute jusqu'à température ambiante.

15. Construction filamentaire et macroporeuse en trois dimensions pouvant être obtenue par le procédé selon l'une quelconque des revendications 1 à 14, qui comprend des filaments qui ont de 1 à 50 %, de préférence de 5 à 30 % de micropores interconnectés, lesdits micropores étant constitués par des pores ayant une taille de pores inférieure ou égale à 100 µm, et lesdits filaments ayant une rugosité moyenne de surface (Ra) supérieure à 4 µm.

16. Construction selon la revendication 15, ladite construction filamentaire et macroporeuse ayant de 50 à 95 %, de préférence de 60 à 85 % de macropores, lesdits macropores étant constitués de pores ayant une taille de pores supérieure à 100 µm.

17. Construction selon l'une quelconque des revendications 15 ou 16, ladite construction filamentaire et macroporeuse ayant un module de compression inférieur à 3 GPa.

18. Construction selon l'une quelconque des revendications 15 à 17, ladite construction filamentaire et macroporeuse ayant une ductilité comprise entre 3 % et 10 %, de préférence entre 5 % et 15 %.

19. Construction selon l'une quelconque des revendications 15 à 18, comprenant des filaments présentant une zone interne et une gaine externe, et la quantité desdits micropores dans lesdits filaments étant différente dans ladite zone interne et dans ladite gaine externe, et de préférence inférieure dans ladite zone interne par rapport à ladite gaine externe.

20. Composition qui comprend une construction selon l'une quelconque des revendications 15 à 19, et qui comprend en outre une protéine morphogénétique osseuse (BMP), des cellules souches, des ostéoblastes, des produits pharmaceutiques et/ou un mélange de ceux-ci.

21. Utilisation d'une construction selon l'une quelconque des revendications 15 à 19, ou d'une composition selon la revendication 20, pour la fabrication d'un produit (bio)médical, tel qu'un implant osseux synthétique ou une greffe osseuse, un échafaudage pour le génie tissulaire, un dispositif de délivrance de médicament.

22. Produit biomédical tel qu'un implant osseux synthétique ou une greffe osseuse, un échafaudage pour le génie tissulaire, un dispositif de délivrance de médicaments comprenant une construction selon l'une quelconque des revendications 15 à 19 ou une composition selon la revendication 20.
